# FASCICULE DE BREVET EUROPEEN

(11) **EP 3 319 520 B1**
(45) Date de publication et mention de la délivrance du brevet: **18.03.2020**
(21) Numéro de dépôt: 16733360.8
(22) Date de dépôt: 21.06.2016
(51) Int. Cl.: A61B 6/00

(54) **CASSETTE RADIOLOGIQUE PORTABLE AVEC MOYEN D'IDENTIFICATION DU PATIENT**
TRAGBARE RADIOLOGISCHE KASSETTE MIT PATIENTENIDENTIFIKATIONSMITTELN
PORTABLE RADIOLOGICAL CASSETTE COMPRISING PATIENT IDENTIFICATION MEANS

(30) Priorité: 08.07.2015 FR 1501445
(43) Date de publication de la demande: 16.05.2018
(73) Titulaire: Trixell, 38430 Moirans (FR)
(72) Inventeur: WIRTH, Thibaut, 38430 Moirans (FR); MATESANZ GARCIA, Beatriz, 78141 Velizy Villacoublay Cedex (FR); DUTIN, Jacky, 38430 Moirans (FR)
(74) Mandataire: Collet, Alain
(86) Numéro de dépôt international: PCT/EP2016/064243
(87) Numéro de publication internationale: WO 2017/005482

(56) Documents cités:
- US-A- 5 757 021
- US-A1- 2009 026 392
- US-A1- 2009 194 695
- US-A1- 2011 073 765

## Description

L'invention se situe dans le domaine de l'imagerie. Elle peut être appliquée à tout type d'imageur, notamment les imageurs à rayons X, visibles, infrarouges. L'invention est explicitée ici dans le domaine de l'imagerie médicale par rayons X, ceci à titre d'exemple et sans perte d'applicabilité aux autres domaines d'imagerie. L'invention concerne une cassette radiologique portable avec moyen d'identification du patient. L'invention concerne également un procédé d'identification d'un patient duquel une radiographie doit être effectuée.

L'invention concerne une cassette radiologique portable destinée à équiper un système radiologique numérique. La cassette comprend un détecteur numérique de rayonnement ionisant permettant de fournir une image fonction du rayonnement reçu.

Un système radiologique comprend une source de rayonnement ionisant, comme par exemple un tube à rayons X, permettant de générer un rayonnement X et une station de base comprenant un système de traitement de l'information permettant de synchroniser le tube à rayon X et le détecteur et permettant aussi de réaliser des traitements d'images comme de présenter à l'opérateur l'image corrigée de tous les défauts inhérents au détecteur et améliorée, par exemple par des traitements de rehaussement de contour. Un objet dont on veut obtenir l'image X est placé entre la source et le détecteur. Un tel système peut être utilisé dans de nombreuses applications telles que par exemple la radiologie médicale et le contrôle non destructif. L'invention peut également être mise en œuvre pour d'autres types de rayonnements à détecter notamment des rayonnements gamma.

Par le passé, les systèmes radiologiques étaient volumineux et peu mobiles. Il était nécessaire de positionner l'objet par rapport au système pour obtenir l'image désirée. Avec l'apparition de détecteurs à l'état solide, le détecteur est devenu moins volumineux et il a été possible de déplacer le détecteur par rapport à un objet restant fixe. Pour la radiologie médicale, on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie.

La cassette mobile comprend essentiellement un détecteur numérique de rayonnement ionisant ayant la forme d'un panneau plat et une carte électronique assurant notamment le pilotage du détecteur numérique. Le détecteur et la carte sont disposés dans un boîtier assurant leur protection mécanique.

En radiographie mobile, par exemple quand la radiographie est faite au lit d'un patient immobilisé, une fois la radiographie effectuée, il est impératif d'associer la radiographie effectuée à ce patient. Et donc de manière générale, chaque radiographie effectuée doit être associée au bon patient.

La radiographie peut se faire au moyen de cassettes dites analogiques de type film/écran ou de cassettes à écran radio-luminescent à mémoire (également connues sous le nom de cassettes ERLM pour son abréviation ou sous le nom de CR pour l'abréviation de l'acronyme anglo-saxon Computed Radiography). La radiographie de ce type de cassette ERLM se fait grâce à un film intégré dans la cassette pouvant stocker une image. La lecture du film est réalisée ultérieurement par un lecteur séparé au niveau d'une station de travail. De ce fait, une cassette ne permet de faire qu'une seule radiographie à la fois. Les opérateurs souhaitant effectuer plusieurs radiographies sur différents patients doivent donc prévoir plusieurs cassettes, ce qui peut générer un risque de mélange des cassettes et de confusion sur l'identification des patients. Il est possible de coller une étiquette avec le nom ou le code du patient sur le dos de chaque cassette, afin de diminuer le risque d'une mauvaise attribution de l'image au patient lors du développement de la radiographie. Les étiquettes peuvent être prises au lit du patient ou être imprimées au préalable. Cette solution n'est néanmoins pas complètement satisfaisante dans un milieu hospitalier. En effet, les radiographies prises au lit des patients se font généralement successivement à l'occasion d'une tournée des chambres. Il faut prévoir plusieurs cassettes, et le risque de confusion sur l'identification du patient demeure.

Une autre solution consiste à générer des codes (par exemple des codes barre). Une cassette reçoit un code barre. L'opérateur note sur la cassette le nom du patient, éventuellement la partie radiographiée. A l'aide d'un lecteur de codes barre, le code est lu et l'image qui sera faite est alors attribuée au patient choisi par l'opérateur. Cette solution implique une lecture du code barre qui se fait après la tournée des chambres, au niveau de la station de travail. Il revient donc à l'opérateur d'ouvrir au niveau de la station de travail le dossier du bon patient avant de lancer la lecture de la cassette. Le risque de confusion entre patients et images est toujours présent et il faut toujours prévoir plusieurs cassettes si plusieurs radiographies sont prévues, ce qui est encombrant.

Comme mentionné précédemment, il existe des cassettes dites numériques sensibles aux rayons X. La cassette numérique n'a pas besoin d'être connectée au générateur de rayons X, elle détecte seule les rayons X et enregistre les images au fur et à mesure de leur prise. Autrement dit, une seule cassette numérique peut contenir plusieurs images. Cette solution a l'avantage pour l'opérateur de ne plus devoir prendre plusieurs cassettes quand il doit faire plusieurs radiographies. Par contre, là encore, le risque de confusion sur l'identification du patient auquel correspond chaque image stockée est réel puisque la cassette numérique contient plusieurs images et il faut pouvoir définir à quel patient correspond chacune des images réalisées et stockées dans la cassette. Une possibilité pour réduire le risque de confusion sur l'identification du patient est d'avoir un code barre attribué au patient qui est lu, avant la radiographie, avec un lecteur de codes barre additionnel par l'opérateur. Cette lecture permet l'ouverture du dossier du patient sur l'ordinateur de l'ensemble de radiologie mobile. Mais une telle solution nécessite donc un système de radiologie mobile comprenant un lecteur de cassette et un ordinateur pour faire l'acquisition de l'image. Cette solution est un mode non-autonome puisqu'il faut prévoir un charriot de transport avec ordinateur et lecteur de cassette. De plus, cette solution n'est pas compatible à une adaptation aux structures préexistantes pour cassettes à films argentiques de dimensions définies par la norme ISO4090.

Il existe une solution consistant à intégrer un petit écran sur la cassette numérique. Ce petit écran permet l'affichage d'un nombre incrémenté à chaque prise d'image. Ce nombre correspond donc au nombre d'images stockées en mémoire dans le détecteur. La radiographie numéro 1 appartient au premier patient, la radiographie numéro 2 appartient au deuxième patient, etc. Ou bien si l'opérateur doit effectuer plusieurs radiographies du même patient, la radiographie numéro 1 peut appartenir à un premier patient, la radiographie numéro 2 peut appartenir au patient numéro 2, la radiographie numéro 3 peut appartenir au même patient numéro 2. Par ailleurs, en plus d'identifier le patient, il est nécessaire d'identifier quelle partie du corps a été radiographiée. Le risque de mélange entre images stockées et patients est important lors du déchargement des images dans la station de travail. En effet, il appartient à l'opérateur de noter manuellement, par exemple sur la fiche du patient ou sur sa propre fiche de travail, le numéro affiché sur le petit écran de la cassette lorsqu'il fait une radiographie d'un patient, et éventuellement la partie du corps radiographiée. Si l'opérateur veut éviter cette source d'erreur due à la notation manuelle du numéro affiché sur la fiche du patient, il peut attribuer au préalable les codes aux patients. Mais cette solution oblige alors l'opérateur à respecter l'ordre de passage ainsi préétabli pour effectuer les radiographies. Autrement dit, si on considère que le numéro affiché est un code d'identification du patient (en établissant au préalable que le numéro 1 est attribué à tel patient, le numéro 2 est attribué à tel autre patient, etc.), l'ordre dans lequel les radiographies doivent être effectuées est imposé, sous peine d'avoir une mauvaise association entre les images stockées et les patients lors du déchargement des images dans la station de travail. US2009/026392 est considéré comme l'état de la technique le plus proche

L'invention vise à annuler tout risque de confusion entre les radiographies de patients, tout en permettant un mode complètement autonome de la cassette numérique, c'est-à-dire sans lecteur portatif additionnel ni d'ordre préétabli de radiographies.

A cet effet, l'invention a pour objet une cassette radiologique portable selon la revendication 1.

Avantageusement, l'image numérisée est stockée dans l'espace mémoire et le code d'identification du patient est écrit dans l'espace mémoire.

Selon un mode de réalisation, le code d'identification du patient est un code-barre, et le dispositif de sélection du code d'identification du patient est un lecteur de code barre.

Selon un autre mode de réalisation, le code d'identification du patient est un QR-code, et le dispositif de sélection du code d'identification du patient est un lecteur de QR-code.

Selon un autre mode de réalisation, l'espace mémoire comprend une première zone et une deuxième zone, et l'image numérisée est stockée dans la première zone de l'espace mémoire et le dispositif de sélection du code d'identification du patient est une interface homme-machine configurée pour qu'un utilisateur puisse choisir le code d'identification dans une liste de patients disponible dans la deuxième zone de l'espace mémoire du détecteur.

Selon un autre mode de réalisation, l'interface homme-machine comprend un écran tactile permettant de sélectionner un patient dans une liste de patients disponible dans la deuxième zone de l'espace mémoire du détecteur.

Selon un autre mode de réalisation, l'interface homme-machine comprend un écran et au moins un bouton, l'écran permettant d'afficher une liste de patients disponible dans la deuxième zone de l'espace mémoire du détecteur et le au moins un bouton permettant de sélectionner un patient dans la liste.

Avantageusement, la cassette radiologique portable comprend des moyens pour consulter une liste de patients sur un serveur extérieur.

Avantageusement, la cassette radiologique portable comprend en outre un écran d'affichage destiné à afficher le code d'identification lu.

L'invention concerne aussi un procédé d'identification d'un patient, soumis au rayonnement ionisant auquel est associé un code d'identification, mettant en œuvre une telle cassette radiologique portable, et comprenant :
- une étape de sélection du code d'identification du patient par le dispositif de sélection du code d'identification,
- une étape d'écriture du code d'identification du patient dans l'espace mémoire du détecteur de la cassette.

L'invention sera mieux comprise et d'autres avantages apparaîtront à la lecture de la description détaillée d'un mode de réalisation donné à titre d'exemple, description illustrée par le dessin joint dans lequel :
- la figure 1 représente schématiquement un exemple de système radiologique mettant en œuvre l'invention,
- la figure 2 représente en vue éclatée un exemple de cassette portable,
- la figure 3 représente schématiquement un premier mode de réalisation d'une cassette selon l'invention,
- la figure 4 représente schématiquement un deuxième mode de réalisation d'une cassette selon l'invention,
- la figure 5 représente les étapes d'un procédé d'identification d'un patient selon l'invention.

Par souci de clarté, les échelles ne sont pas respectées. De plus, les mêmes éléments porteront les mêmes repères dans les différentes figures. L'invention est décrite dans le domaine de l'imagerie médicale par rayons X mais elle trouve application dans tout autre domaine à rayonnement ionisant, par exemple les rayons gamma.

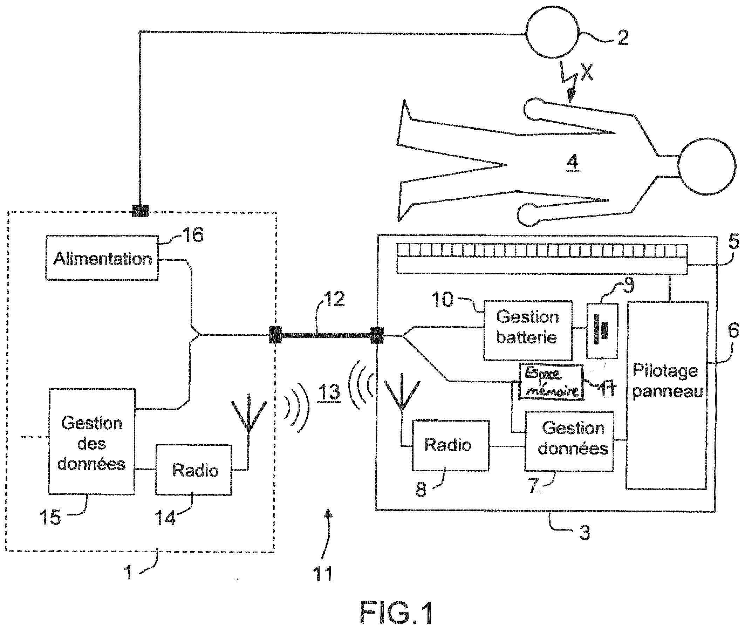

La figure 1 représente schématiquement un exemple de système radiologique mettant en œuvre l'invention. La figure 1 représente un système radiologique destiné à une utilisation médicale. Le système comporte une station de base fixe 1 un générateur de rayonnement X 2 et un détecteur de rayonnement sous forme d'une cassette portable 3. La cassette permet d'obtenir une image d'un patient 4 traversé par le rayonnement X issu du générateur de rayonnement 2. La cassette 3 comporte un détecteur numérique réalisé sous forme d'un panneau plat 5 relié à un module de pilotage 6 permettant de lire l'image obtenue par le panneau plat 5 et de la numériser au travers d'un convertisseur analogique numérique. La cassette mobile 3 comporte également un module de gestion de données 7, un module radio 8, une batterie 9 et un module de gestion de la batterie 10. La cassette mobile comprend un espace mémoire 17 destiné à stocker l'image numérisée.

La station de base comporte un module radio 14, un module de gestion de données 15 et une alimentation 16.

Des moyens de communication 11 entre la cassette 3 et la station de base 1 permettent de transférer des données telles que l'image entre la cassette 3 et la station de base 1. Les données peuvent circuler soit de la station de base 1 vers la cassette 3, soit de la cassette 3 vers la station de base 1. Vers la cassette 3, il s'agit par exemple d'informations de commande du panneau plat 5 et vers la station de base 1, les données comportent par exemple des images réalisées par le panneau plat 5.

Les moyens de communication peuvent comprendre une liaison filaire amovible 12 et/ou une liaison sans fil 13. Les deux liaisons 12 et 13 sont susceptibles toutes deux de transférer les données. Les deux modules radio 8 et 14 permettent d'échanger les données entre la station de base 1 et la cassette 3. Le module de gestion de données 7 de la cassette 3 permet d'aiguiller les données reçues ou en provenance du module de pilotage 6 vers l'une des liaisons 12 ou 13. De même, dans la station de base 1, le module de gestion de données 15 permet d'aiguiller les données reçues ou en provenance d'une des liaisons 12 ou 13. L'alimentation 16 fournit l'énergie électrique nécessaire au fonctionnement des différents modules de la station de base 1 ainsi que de la cassette 3.

L'alimentation de la cassette 3 se fait par l'intermédiaire de la liaison filaire 12 ou de la batterie 9. Avantageusement, le système comporte des moyens pour recharger la batterie 9. Plus précisément, le module de gestion de la batterie 10 mesure la charge de la batterie 9 et provoque sa recharge en cas de besoin.

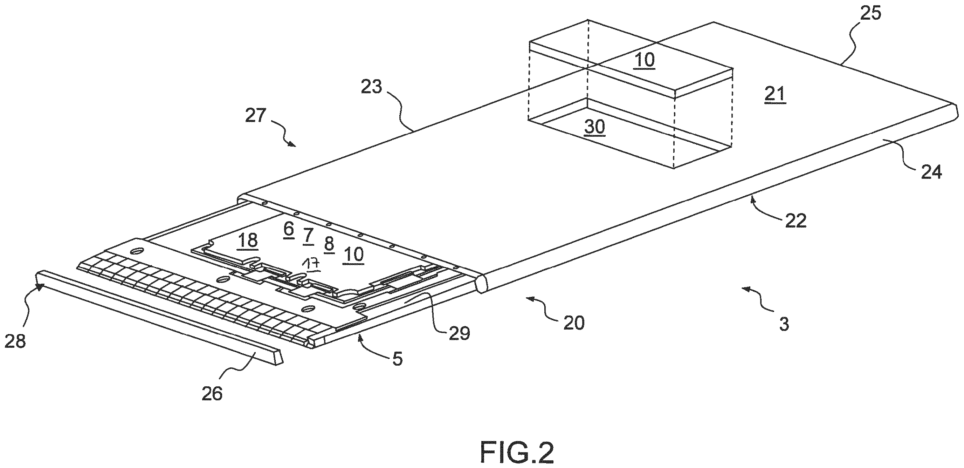

La figure 2 représente un exemple de cassette portable en vue éclatée. La cassette comprend un boitier 20 ayant une forme essentiellement parallélépipédique dans lequel sont disposés le détecteur numérique 5, et une carte électronique 18 assurant la gestion du détecteur 5. Pour se référer à la figure 1, la carte électronique 18 comprend par exemple le module de pilotage 6, le module de gestion de données 7, le module radio 8, et le module de gestion de la batterie 10. Ces quatre modules ne sont donnés qu'à titre d'exemple. Ils ne sont pas obligatoires pour la mise en œuvre de l'invention. La batterie 9 est disposée à l'extérieur du boitier 20 afin de faciliter un éventuel remplacement. La carte électronique 18 comprend aussi l'espace mémoire 17.

Le boitier 20 possède six faces principales 21 à 26 délimitant la forme parallélépipédique. Les six faces sont parallèles deux à deux. Le détecteur 5, sous forme d'un panneau plat, possède une surface de détection de rayonnement proche de celle des deux plus grandes faces 21 et 22. Les faces parallèles 25 et 26 sont les deux plus petites faces du boitier 20.

Le boitier 20 comprend par exemple une enveloppe 27 réalisée dans une pièce mécanique monobloc formant les cinq faces 21 à 25 de la forme essentiellement parallélépipédique dont les deux plus grandes faces 21 et 22. Le boitier 20 peut comprendre en outre un bouchon 28 permettant d'obturer la face 26 de la forme essentiellement parallélépipédique. Alternativement, le bouchon 28 peut obturer la face 23 ou la face 24.

Le fait de réaliser une pièce monobloc sur cinq faces permet de rigidifier fortement le boitier. Plus particulièrement, les trois plus petites faces 23, 24 et 25 (respectivement 26, 23, 25 ou 26, 24, 25 selon la face obturée par le bouchon 28) ceinturent le boitier 20 selon deux directions perpendiculaires, ce qui augmente la rigidité du boitier 20 en torsion autour d'axes parallèles aux deux plus grandes faces 21 et 22.

Les différents éléments disposés à l'intérieur du boitier 20 sont solidaires les uns des autres et sont glissés dans l'enveloppe 27 par la face 26 (respectivement 23 ou 24 selon la face obturée par le bouchon 28) dans un mouvement de translation perpendiculairement à cette face.

Il est également possible d'avoir une enveloppe 27 réalisée dans une pièce mécanique monobloc formant les quatre faces 21 à 24. Dans ce cas, le boitier 20 comprend deux bouchons 28 et 28' permettant d'obturer les faces 26 et 25. Le bouchon 28 peut être un bouchon dit mobile, et le bouchon 28' peut être un bouchon dit fixe. Les bouchons peuvent être un support à certaines fonctionnalités comme la connectique. Par ailleurs, l'invention est décrite dans la configuration selon laquelle les bouchons 28 et 28' obturent les faces 26 et 25. Il est bien évident que l'invention peut s'appliquer à d'autres configurations selon lesquelles les bouchons 28 et 28' obturent deux faces parmi les faces 23, 24, 25, 26. Et plus généralement, l'invention peut s'appliquer avec un seul bouchon 28 obturant une de ces faces.

La batterie 10 est logée à l'intérieur du volume parallélépipédique formée par le boitier 20. La batterie 10 est logée par l'extérieur de la cassette 3 dans un embrèvement 30 réalisée dans la face 21. La face 22, opposée à la face 21, est destinée à être traversée par le rayonnement ionisant à détecter. Le détecteur numérique 5 est disposé à l'intérieur du boitier 20 du coté de la face 22.

Par le passé, la radiologie médicale utilisait des films argentiques qui étaient manipulés dans des cassettes. La norme ISO 4090 a défini les dimensions des cassettes enveloppant les films argentiques. L'épaisseur des cassettes, définie par la norme, est comprise entre 13 et 16 mm. Avantageusement, la cassette répond, quant à ses dimensions, aux exigences de la norme ISO 4090. Plus particulièrement, l'épaisseur hors tout de la cassette 3 mesurée entre les deux plus grandes faces 21 et 22 est inférieure à 16 mm. Ceci permet d'utiliser les moyens de rangements de cassettes argentiques pour une cassette numérique 3.

Comme expliqué précédemment, une seule cassette numérique peut contenir plusieurs images. Cette solution a l'avantage pour un opérateur de ne plus devoir prendre plusieurs cassettes quand il doit faire plusieurs radiographies, par exemple lors d'une tournée des chambres pour faire une ou plusieurs radiographies de plusieurs patients. Néanmoins, le fait qu'une cassette numérique puisse contenir plusieurs images entraîne un risque de confusion sur l'identification du patient auquel correspond chaque image stockée. En effet, comme la cassette numérique contient plusieurs images, il faut pouvoir définir à quel patient correspond chacune des images réalisées et stockées dans la cassette.

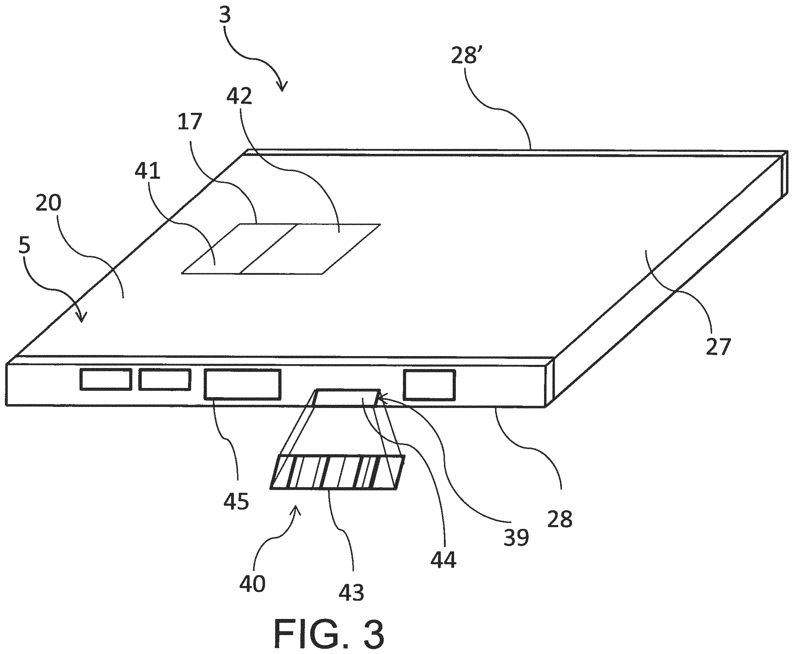

La figure 3 représente schématiquement un premier mode de réalisation d'une cassette selon l'invention. La cassette 3 est identique à la cassette présentée sur la figure 2 et peut être mise en œuvre de la même manière que la cassette présentée sur la figure 1. La cassette radiologique portable 3 comprend un boitier 20, un détecteur numérique d'un rayonnement ionisant incident sous forme d'un panneau plat, le détecteur 5 étant positionné dans le boitier 20 et comprenant un espace mémoire 17, et destiné à générer une image numérisée d'un patient soumis au rayonnement ionisant auquel est associé un code d'identification 40, l'image numérisée étant stockée dans l'espace mémoire 17. Selon l'invention, la cassette 3 comprend un dispositif de sélection 39 du code d'identification 40 du patient destiné à écrire le code d'identification 40 du patient dans l'espace mémoire 17. Ainsi, à chaque image stockée dans l'espace mémoire 17 est associé un code d'identification 40 du patient. L'invention permet à l'opérateur de pouvoir faire les radiographies des patients dans un ordre quelconque non prédéfini préalablement, puisqu'au moment où il réalise l'image d'un patient, le code d'identification 40 du patient est également enregistré dans l'espace mémoire 17 de la cassette 3. L'enregistrement du code d'identification 40 du patient dans l'espace mémoire 17 permet d'annuler tout risque de confusion entre les radiographies de patients. De plus, un ordre préétabli de radiographies n'est pas nécessaire, ce qui décontraint l'opérateur dans sa tournée des chambres. Comme la cassette 3 comprend le dispositif de sélection 39, la cassette numérique fonctionne en mode complètement autonome, c'est-à-dire sans lecteur portatif additionnel, ce qui permet de réduire l'encombrement de l'ensemble de radiographie mobile. De plus, la cassette 3 est compatible à une adaptation aux structures préexistantes pour cassettes à films argentiques de dimensions définies par la norme ISO4090.

En d'autres termes, le détecteur peut générer une pluralité d'images numérisées de patients soumis au rayonnement ionisant. Un code d'identification est associé à chacun des patients, et chaque image de la pluralité d'images numérisées d'un patient étant stockée dans l'espace mémoire. Par ailleurs, le dispositif de sélection du code d'identification du patient peut écrire le code d'identification du patient dans l'espace mémoire. Autrement dit, l'invention permet de générer des images, par exemple trois images nommées I1, I2, I3. Les images I1, I2, I3 sont stockées dans l'espace mémoire de la cassette. Et à chacune de ces images correspond un code, par exemple C1 pour l'image I1, C2 pour l'image I2, C3 pour l'image I3. Si le code correspond au nom d'un patient et que les images I1 et I2 sont issues du même patient, alors les images I1 et I2 peuvent avoir toutes les deux le même code, par exemple P1 et l'image I3 issue d'un autre patient peut avoir le code P2. L'espace mémoire de la cassette est donc une zone dans laquelle on peut sauvegarder une information associée à au moins une des images générées, l'information pouvant être un code d'identification, un nom d'un patient, un numéro d'assurance sociale d'un patient et/ou une partie de corps radiographié. Bien entendu, l'espace mémoire peut contenir une combinaison d'informations.

Plus précisément, l'espace mémoire 17 peut être scindé en deux parties et comprendre une première zone 41 et une deuxième zone 42. L'image numérisée est stockée dans la première zone 41 de l'espace mémoire 17 et le code d'identification 40 du patient peut être écrit dans la première zone 41 de l'espace mémoire. Cette répartition de l'espace mémoire 17 peut permettre d'écrire le code d'identification 40 du patient sur l'image numérisée. Autrement dit, le code d'identification 40 du patient peut être directement intégré dans l'en-tête de l'image stockée dans la mémoire du détecteur 5.

Sur la figure 3, l'enveloppe 27 est réalisée dans une pièce mécanique monobloc formant les quatre faces. Et le boitier 20 comprend deux bouchons 28 et 28' permettant d'obturer les faces. Dans ce premier mode de réalisation, le bouchon 28 est un bouchon mobile et le bouchon 28' est un bouchon fixe.

Le code d'identification 40 du patient peut être un code et le dispositif de sélection 39 du patient est un lecteur de code. Notamment, le code d'identification 40 du patient peut être un code-barre 43 et le dispositif de sélection 39 du patient est un lecteur de code-barre 44. De même, le code d'identification 40 du patient peut être un QR-code (QR étant l'abréviation du terme anglo-saxon Quick Response signifiant que le contenu du code peut être décodé rapidement après avoir été lu) et le dispositif de sélection 39 du patient est un lecteur de QR-code. Le QR-code présente l'avantage de pouvoir stocker plus d'informations qu'un code-barre. De manière plus générale, l'invention peut s'appliquer avec tout autre type de code pouvant être lu par un lecteur associé à ce type de code. L'invention s'applique également à un dispositif de sélection 39 tel qu'un lecteur optique (de type caméra) pouvant lire n'importe quelle information (code barre, QR code et tout autre symbole ou lettre).

Sur la figure 3, le lecteur de code-barre 44 est positionné sur le bouchon mobile 28. La cassette 3 selon l'invention peut aussi comprendre un écran d'affichage 45 destiné à afficher le code d'identification 40 lu. Cet écran d'affichage 45 permet notamment à l'opérateur d'assurer un contrôle supplémentaire de l'identification du patient. Les positions des éléments sur la figure 3 sont données à titre d'exemple. Il est bien entendu que l'invention ne se limite pas à ces positions. Par exemple, l'écran d'affichage 45 peut également être positionné sur le bouchon fixe 28' ou même sur la face arrière de la cassette 3.

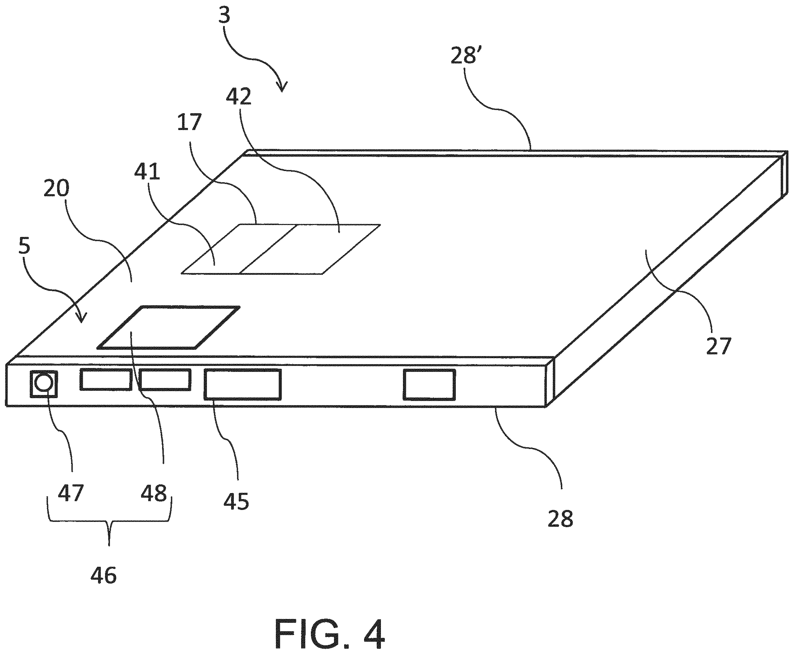

La figure 4 représente schématiquement un deuxième mode de réalisation d'une cassette selon l'invention. La cassette 3 représentée sur la figure 4 est identique à la cassette représentée sur la figure 3 à l'exception du dispositif de sélection 39 du code d'identification 40 du patient. Sur la figure 4, le dispositif de sélection 39 du code d'identification 40 du patient est une interface homme-machine 46, aussi connue sous son abréviation IHM, configurée pour qu'un utilisateur puisse choisir le code d'identification 40 dans une liste de patients disponible dans la deuxième zone 42 de l'espace mémoire 17 du détecteur 5.

L'interface homme-machine 46 peut comprendre un écran 48 et au moins un bouton 47, l'écran 48 permettant d'afficher une liste de patients disponible dans la deuxième zone 42 de l'espace mémoire 17 et le bouton 47 permettant de sélectionner un patient dans la liste. Plus exactement, l'écran 48 permet d'afficher une liste de patients disponible dans la deuxième zone 42 de l'espace mémoire 17 ou une liste de codes d'identification 40 également disponible dans la deuxième zone 42 de l'espace mémoire 17.

Alternativement, l'écran 48 peut être un écran multitouche permettant de proposer différents affichages au choix, notamment une prévisualisation de la radiographie ou tout autre affichage d'information que l'opérateur peut souhaiter.

Alternativement, l'écran 48 peut être un écran tactile permettant directement (sans bouton supplémentaire) de sélectionner un patient dans une liste de patients disponible dans la deuxième zone 42 de l'espace mémoire 17 du détecteur. De même, l'écran 48 permet d'afficher une liste de patients disponible dans la deuxième zone 42 de l'espace mémoire 17 ou une liste de codes d'identification 40 des patients également disponible dans la deuxième zone 42 de l'espace mémoire 17. Dans ce deuxième mode de réalisation, la liste de patients disponible et/ou leur code d'identification 40 dans la deuxième zone 42 de l'espace mémoire 17 du détecteur a été téléchargée au préalable.

Par exemple, avant de faire sa tournée des chambres, l'opérateur télécharge dans la cassette 3 la liste de tous les patients dont il souhaite faire une radiographie. Pendant sa tournée, l'opérateur peut ainsi choisir l'ordre dans lequel il va radiographier les patients. Ceci présente l'avantage de ne pas imposer l'ordre de passage qui serait déterminé par une liste préétablie. Ainsi, si un patient est indisponible (par exemple, endormi ou recevant des soins), l'opérateur peut faire la radiographie ultérieurement, en fin de tournée par exemple, sans générer pour autant une confusion sur les images numérisées puisqu'à chaque image sera attribué le bon patient auquel l'image correspond.

Avantageusement, la cassette 3 selon l'invention peut comprendre des moyens pour consulter une liste de patients sur un serveur extérieur. Par exemple, dans un milieu hospitalier disposant d'un serveur de données accessible en connexion sans fil, les moyens pour consulter la liste de patients peuvent être des moyens de communication comprenant la liaison sans fil 13, le module de gestion de données 7 et le module radio 8. La liste de patients peut ainsi être enregistrée sur le serveur de données du milieu hospitalier. L'avantage de cette configuration est de permettre à l'opérateur de ne pas devoir télécharger préalablement à sa tournée des chambres la liste des patients dont il souhaite faire une radiographie. Grâce à la connexion sans fil, l'opérateur a accès, via l'interface homme-machine 46, à la liste de patients sur le serveur de données. Après avoir choisi le patient, l'opérateur peut faire la radiographie. Le code d'identification 40 du patient peut alors être intégré dans l'en-tête de l'image stockée dans la mémoire du détecteur. Ensuite, l'opérateur peut faire d'autres radiographies d'autres patients. Lors du déchargement des images stockées dans un serveur du milieu médical, chacune des images peut être automatiquement affectée au dossier respectif des patients.

Comme déjà mentionné dans la description, le principal avantage de l'invention est de permettre un mode complètement autonome de la cassette numérique, sans risque de confusion entre les radiographies de patients, sans lecteur portatif additionnel ni d'ordre préétabli de radiographies.

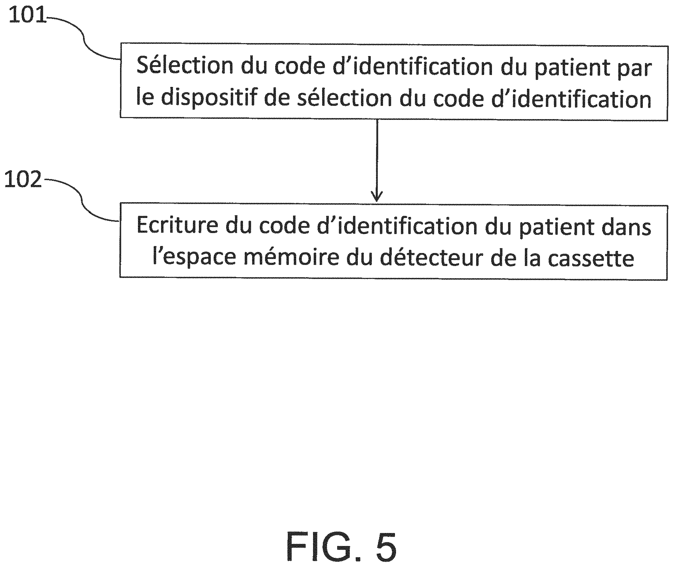

La figure 5 représente les étapes d'un procédé d'identification d'un patient duquel une radiographie doit être effectuée. Selon l'invention, le procédé d'identification du patient comprend une étape 101 de sélection du code d'identification 40 du patient par le dispositif de sélection 39 du code d'identification 40, une étape 102 d'écriture du code d'identification 40 du patient dans l'espace mémoire 17 du détecteur 5 de la cassette 3.

L'invention a été décrite dans un cas d'application à la radiographie en milieu médical. Il est bien évident que l'invention trouve aussi application dans d'autres domaines et/ou avec d'autres rayonnements ionisants.

## Revendications

1. Cassette radiologique portable (3) comprenant :
• un boitier (20),
• un détecteur numérique d'un rayonnement ionisant incident sous forme d'un panneau plat (5), le détecteur étant positionné dans le boitier et comprenant un unique espace mémoire (17), et destiné à générer une pluralité d'images numérisées de patients soumis au rayonnement ionisant, un code d'identification (40) étant associé à chacun des patients, chaque image de la pluralité d'images numérisées des patients étant stockée dans l'unique espace mémoire (17),
**caractérisée en ce qu'**elle comprend un dispositif de sélection (39) du code d'identification (40) des patients destiné à écrire le code d'identification (40) des patients dans l'unique espace mémoire (17), de sorte à associer dans l'unique espace mémoire (17) chaque image de la pluralité d'images à un patient.

2. Cassette radiologique portable (3) selon la revendication précédente, **caractérisée en ce que** la pluralité d'images numérisées est stockée dans l'unique espace mémoire (17) et **en ce que** le code d'identification (40) des patients est écrit dans l'unique espace mémoire (17).

3. Cassette radiologique portable (3) selon l'une des revendications 1 ou 2, le code d'identification (40) des patients étant un code-barre (43), **caractérisée en ce que** le dispositif de sélection (39) du code d'identification (40) des patients est un lecteur de code barre (44).

4. Cassette radiologique portable (3) selon l'une des revendications 1 ou 2, le code d'identification (40) des patients étant un QR-code, **caractérisée en ce que** le dispositif de sélection (39) du code d'identification (40) des patients est un lecteur de QR-code.

5. Cassette radiologique portable (3) selon l'une des revendications 1 ou 2, l'unique espace mémoire (17) comprenant une première zone (41) et une deuxième zone (42), **caractérisée en ce que** la pluralité d'images numérisées est stockée dans la première zone (41) de l'unique espace mémoire (17) et **en ce que** le dispositif de sélection (39) du code d'identification (40) des patients est une interface homme-machine (46) configurée pour qu'un utilisateur puisse choisir le code d'identification (40) dans une liste de patients disponible dans la deuxième zone (42) de l'unique espace mémoire (17) du détecteur (5).

6. Cassette radiologique portable selon la revendication 5, **caractérisée en ce que** l'interface homme-machine (46) comprend un écran tactile (48) permettant de sélectionner un patient dans une liste de patients disponible dans la deuxième zone (42) de l'unique espace mémoire (17) du détecteur (5).

7. Cassette radiologique portable (3) selon la revendication 5, **caractérisée en ce que** l'interface homme-machine (46) comprend un écran (48) et au moins un bouton (47), l'écran (48) permettant d'afficher une liste de patients disponible dans la deuxième zone (42) de l'unique espace mémoire (17) du détecteur (5) et le au moins un bouton (47) permettant de sélectionner un patient dans la liste.

8. Cassette radiologique portable (3) selon l'une des revendications 6 ou 7, **caractérisée en ce qu'**elle comprend des moyens pour consulter une liste de patients sur un serveur extérieur.

9. Cassette radiologique portable (3) selon l'une des revendications précédentes, **caractérisée en ce qu'**elle comprend en outre un écran d'affichage (45) destiné à afficher le code d'identification (40) lu.

10. Procédé d'identification d'un patient, soumis au rayonnement ionisant auquel est associé un code d'identification (40), mettant en œuvre une cassette radiologique portable selon l'une des revendications précédentes, **caractérisé en ce qu'**il comprend :
• une étape (101) de sélection du code d'identification (40) du patient par le dispositif de sélection (39) du code d'identification (40),
• une étape (102) d'écriture du code d'identification (40) du patient dans l'unique espace mémoire du détecteur de la cassette.

## Patentansprüche

1. Tragbare radiologische Kassette (3), die Folgendes umfasst:
• ein Gehäuse (20),
• einen digitalen Detektor für einfallende ionisierende Strahlung in Form einer flachen Platte (5), wobei der Detektor in dem Gehäuse positioniert ist und einen einzigen Speicherplatz (17) umfasst und zum Erzeugen einer Vielzahl von digitalisierten Bildern von Patienten bestimmt ist, die der ionisierenden Strahlung ausgesetzt werden, wobei ein Identifikationscode (40) mit jedem der Patienten assoziiert ist, wobei jedes Bild der Vielzahl von digitalisierten Bildern der Patienten an dem einzigen Speicherplatz (17) gespeichert wird,
**dadurch gekennzeichnet, dass** sie eine Vorrichtung (39) zum Auswählen des Identifikationscode (40) von Patienten umfasst, bestimmt zum Schreiben des Identifikationscode (40) der Patienten auf den einzigen Speicherplatz (17), um jedes Bild der Vielzahl von Bildern in dem einzigen Speicherplatz (17) mit einem Patienten zu assoziieren.

2. Tragbare radiologische Kassette (3) nach dem vorherigen Anspruch, **dadurch gekennzeichnet, dass** die Vielzahl von digitalisierten Bildern auf dem einzigen Speicherplatz (17) gespeichert wird, und dadurch, dass der Identifikationscode (40) der Patienten auf den einzigen Speicherplatz (17) geschrieben wird.

3. Tragbare radiologische Kassette (3) nach Anspruch 1 oder 2, wobei der Identifikationscode (40) der Patienten ein Barcode (43) ist, **dadurch gekennzeichnet, dass** die Vorrichtung (39) zum Auswählen des Identifikationscode (40) der Patienten ein Barcodeleser (44) ist.

4. Tragbare radiologische Kassette (3) nach Anspruch 1 oder 2, wobei der Identifikationscode (40) der Patienten ein QR-Code ist, **dadurch gekennzeichnet, dass** die Vorrichtung (39) zum Auswählen des Identifikationscode (40) der Patienten ein QR-Code-Leser ist.

5. Tragbare radiologische Kassette (3) nach Anspruch 1 oder 2, wobei der einzige Speicherplatz (17) eine erste Zone (41) und eine zweite Zone (42) umfasst, **dadurch gekennzeichnet, dass** die Vielzahl von digitalisierten Bildern in der ersten Zone (41) des einzigen Speicherplatzes (17) gespeichert wird, und dadurch, dass die Vorrichtung (39) zum Auswählen des Identifikationscode (40) der Patienten eine Mensch-Maschine-Schnittstelle (46) ist, so konfiguriert, dass ein Benutzer den Identifikationscode (40) aus einer in der zweiten Zone (42) des einzigen Speicherplatzes (17) des Detektors (5) verfügbaren Liste von Patienten auswählen kann.

6. Tragbare radiologische Kassette (3) nach Anspruch 5, **dadurch gekennzeichnet, dass** die Mensch-Maschine-Schnittstelle (46) einen Touchscreen (48) umfasst, der es zulässt, einen Patienten aus einer in der zweiten Zone (42) des einzigen Speicherplatzes (17) des Detektors (5) verfügbaren Patientenliste auszuwählen.

7. Tragbare radiologische Kassette (3) nach Anspruch 5, **dadurch gekennzeichnet, dass** die Mensch-Maschine-Schnittstelle (46) einen Bildschirm (48) und wenigstens eine Taste (47) umfasst, wobei der Bildschirm (48) das Anzeigen einer in der zweiten Zone (42) des einzigen Speicherplatzes (17) des Detektors (5) verfügbaren Liste von Patienten zulässt, wobei die wenigstens eine Taste (47) das Auswählen eines Patienten aus der Liste zulässt.

8. Tragbare radiologische Kassette (3) nach Anspruch 6 oder 7, **dadurch gekennzeichnet, dass** sie Mittel zum Konsultieren einer Patientenliste auf einem externen Server umfasst.

9. Tragbare radiologische Kassette (3) nach einem der vorherigen Ansprüche, **dadurch gekennzeichnet, dass** sie ferner einen Anzeigeschirm (45) zum Anzeigen des gelesenen Identifikationscode (40) umfasst.

10. Verfahren zum Identifizieren eines Patienten, der der ionisierenden Strahlung ausgesetzt wird, mit dem ein Identifikationscode (40) assoziiert ist, unter Nutzung einer tragbaren radiologischen Kassette nach einem der vorherigen Ansprüche, **dadurch gekennzeichnet, dass** es Folgendes beinhaltet:
• einen Schritt (101) des Auswählens des Identifikationscode (40) des Patienten mit der Vorrichtung (39) zum Auswählen des Identifikationscode (40),
• einen Schritt (102) des Schreibens des Identifikationscode (40) des Patienten in den einzigen Speicherplatz des Detektors der Kassette.

## Claims

1. A portable radiological cassette (3) comprising:
• a housing (20),
• a digital detector of incident ionizing radiation, taking the form of a flat panel (5), the detector being positioned in the housing and comprising a unique memory space (17), and being intended to generate a plurality of digital images of patients exposed to the ionizing radiation, an identification code (40) being associated with each of the patients, each image of the plurality of digital images of the patients being stored in the unique memory space (17);
**characterized in that** it comprises a device (39) for selecting the identification code (40) of the patients, which is intended to write the identification code (40) of the patients in the unique memory space (17), so as to associate in the unique memory space (17) each image of the plurality of images to a patient.

2. The portable radiological cassette (3) as claimed in the preceding claim, **characterized in that** the plurality of digital images is stored in the unique memory space (17) and **in that** the identification code (40) of the patients is written in the unique memory space (17).

3. The portable radiological cassette (3) as claimed in either of claims 1 and 2, the identification code (40) of the patients being a barcode (43), **characterized in that** the device (39) for selecting the identification code (40) of the patients is a barcode reader (44).

4. The portable radiological cassette (3) as claimed in either of claims 1 and 2, the identification code (40) of the patients being a QR code, **characterized in that** the device (39) for selecting the identification code (40) of the patients is a QR-code reader.

5. The portable radiological cassette (3) as claimed in either of claims 1 and 2, the unique memory space (17) comprising a first zone (41) and a second zone (42), **characterized in that** the plurality of digital images is stored in the first zone (41) of the unique memory space (17) and **in that** the device (39) for selecting the identification code (40) of the patients is a human-machine interface (46) configured so that a user can choose the identification code (40) from a list of patients that is available in the second zone (42) of the unique memory space (17) of the detector (5).

6. The portable radiological cassette as claimed in claim 5, **characterized in that** the human-machine interface (46) comprises a touchscreen (48) allowing a patient to be selected from a list of patients that is available in the second zone (42) of the unique memory space (17) of the detector (5).

7. The portable radiological cassette (3) as claimed in claim 5, **characterized in that** the human-machine interface (46) comprises a screen (48) and at least one button (47), the screen (48) allowing a list of patients that is available in the second zone (42) of the unique memory space (17) of the detector (5) to be displayed and the at least one button (47) allowing a patient to be selected from the list.

8. The portable radiological cassette (3) as claimed in either of claims 6 and 7, **characterized in that** it comprises means for consulting a list of patients on a remote server.

9. The portable radiological cassette (3) as claimed in one of the preceding claims, **characterized in that** it furthermore comprises a display screen (45) intended to display the identification code (40) read.

10. A method for identifying a patient exposed to ionizing radiation with whom an identification code (40) is associated, implementing a portable radiological cassette as claimed in one of the preceding claims, **characterized in that** it comprises:
• a step (101) of selecting the identification code (40) of the patient with the device (39) for selecting the identification code (40),
• a step (102) of writing the identification code (40) of the patient in the unique memory space of the detector of the cassette.
